# EUROPEAN PATENT SPECIFICATION

(11) **EP 3 125 704 B1**
(45) Date of publication and mention of the grant of the patent: **15.11.2017**
(21) Application number: 15715201.8
(22) Date of filing: 27.03.2015
(51) Int. Cl.: A24D 3/14, A61K 36/81, A61K 41/00, A61K 9/00, A61K 31/7016

(54) **HOMEOPATHIC PROCEDURE FOR THE NEUTRALIZATION OF DAMAGE FROM CIGARETTE SMOKE AND "NEUTRALIZED" CIGARETTE OBTAINED THEREFROM**
HOMÖOPATHISCHES VERFAHREN ZUR NEUTRALISIERUNG VON SCHÄDEN AUS ZIGARETTENRAUCH UND AUF DIESE WEISE ERHALTENE NEUTRALISIERTE ZIGARETTE
PROTOCOLE HOMÉOPATHIQUE POUR LA NEUTRALISATION DE DOMMAGES DUS LA FUMÉE DE CIGARETTE ET CIGARETTE "NEUTRALISÉE" OBTENUE À PARTIR DE CELUI-CI

(30) Priority: 31.03.2014 IT MI20140556
(43) Date of publication of application: 08.02.2017
(73) Proprietor: Colombo, Claudio, 27020 Travacò Siccomario (Pavia) (IT)
(72) Inventor: Colombo, Claudio, 27020 Travacò Siccomario (Pavia) (IT)
(74) Representative: Eterno, Enrico
(86) International application number: PCT/EP2015/056792
(87) International publication number: WO 2015/150284

(56) References cited:
- US-A- 3 144 024
- US-A- 5 060 669

## Description

The present invention relates to homeopathic cigarettes able to neutralize the damage from smoke and to the procedure for obtaining them.

More particularly it relates to cigarettes provided with filter apt to neutralize the harmful effects of active smoking on the smoker, and again more particularly to cigarettes provided with a normal filter yet soaked with a specific homeopathic preparation (e.g. hydro-alcoholic homeopathic solution) able to neutralize the damage from smoke so as to obtain "neutralized" cigarettes. Cigarette filters treated with a tobacco extract for the purpose of improving taste or flavour of cigarette smoke are known e.g. from US patent publications US3144024 and US5060669. There are currently no commercially available remedies for the neutralization of damage from smoke nor even less so homeopathic remedies for the same purpose.

Drugs instead exist for giving up smoking or electronic cigarettes, both of which have disadvantages from the point of view of health of the user.

The need is therefore very much felt in the sector of cigarettes for the availability of remedies for the neutralization of the damage from cigarette smoke.

There are over 1 billion smokers in the world who smoke approximately 6 thousand billion cigarettes a year: therefore, on average, each smoker consumes approximately 6.5 kg/year of tobacco, with average consumption of 1,600 cigarettes/year.

While it is true that smoking causes well-documented damage, it is also true that smoking can be useful for relieving personal stresses and at times the damage from the cigarette can even be preferable to that of stress.

Cigarette smoke is not composed of nicotine alone: over 2,000 substances have been detected, deriving from the simultaneous combustion of the tobacco and of the preservatives, among which we can cite:
- tar: produced by incomplete combustion and condensation of high-boiling-point substances;
- VOCs - volatile organic compounds including the carcinogen benzene;
- APHs - aromatic polycyclic hydrocarbons coming from the combustion both of the paper and of the tobacco, comprising the carcinogens benzopyrenes;
- fine particles, mainly the carbonaceous particulate, also deriving from the incomplete combustion, direct source of cardiovascular problems and substrate whereon some APHs are adsorbed;
- nicotine, contained in the leaves of the tobacco plant and volatilized with heat;
- carbon monoxide produced by the incomplete combustion;
- irritating substances: primarily aldehydes: acrolein and acetaldehyde, the result of incomplete combustion yet at a greater level of oxidation and irritating to the mucous membranes; formaldehyde is a carcinogen; and then carboxylic organic acids: formic acid, phenols such as phenol and cresols;
- amines and ammonia;
- non-aromatic hydrocarbons of various types (mainly alkanes C₁₂-C₁₅) which contribute to attacking the protective lipid layer of organs and cells, as preparation for attack by other compounds;
- other oxidating substances.

The effects of smoking on health show high, statistically significant rates of serious medical problems among smokers. Smoking is considered a factor encouraging the onset of some pathologies, mainly affecting the respiratory apparatus and the cardiovascular apparatus, and in developed countries is considered a primary cause in avoidable deaths.

Moreover smoke is classified as a known carcinogen for humans by the World Health Organisation and some of its components, above all benzene and benzopyrene, are also known carcinogens.

On the basis of evidence smoking is therefore definitely linked to the onset of tumoural pathologies of various kinds, lung cancer in primis in terms of occurrence and mortality.

The harmful substances of smoke which can directly damage the respiratory apparatus, irritate and encourage further damage, yet also indirectly the rest of the body, are essentially produced by the incomplete combustion of tobacco and paper, and substances volatilized due to the relatively high temperature.

The object of the present invention is that of overcoming, at least in part, the disadvantages of the prior art by providing a remedy for the neutralization of the damage from cigarette smoke.

These and other objects are achieved by the cigarette in accordance with the invention having the features listed in the appended independent claim 1.

Advantageous embodiments of the invention are disclosed by the dependent claims.

An object of the present invention relates to a cigarette having a filter soaked with a specific homeopathic preparation, in particular soaked with a homeopathic solution deriving from a mother tincture obtained from the maceration of cigarette tobacco and paper, where the mixture of paper and tobacco constitutes the "homeopathic active ingredient".

More particularly said filter is soaked with this homeopathic solution through the system of "triple impregnation" already known in the art.

Homeopathy is based on the principles formulated by the German doctor Samuel Hahnemann towards the end of the eighteenth century: the basis is the "principle of similarity of the drug" (*similia similibus curantur*), concept whereby the appropriate remedy for a certain illness would be given by that substance which, in a healthy person, induces symptoms similar to those observed in the sick person. This substance, also referred to as "homeopathic active ingredient", once identified, is administered to the patient in a strongly diluted quantity. Its two main theoretical bases are the law of similars (*similia similibus curantur*) and that of the use of infinitesimal doses of the remedies appropriately dynamized via a procedure known as "succussion" (shaking).

Subsequently Hugo Paul Friedrich Schultz claimed that toxins can have an opposite effect if administered in small doses compared to the usual ones, basing on studies performed on yeasts.

In the same period the doctor Rudolph Amdt claimed that weak stimuli slightly accelerate vital activity while strong stimuli suppress it: the aforesaid observations were formulated in a single law, known as that of Arndt-Schultz, which represents one of the oldest laws of homeopathic pharmacology.

Homeopathy therefore begins with small doses and moves towards progressively higher dilutions to stimulate the natural electromagnetic forces of the body. One of the fundamental principles of homeopathic medicine is that a cure for a disease can be obtained using a high dilution of a substance which is similar to yet different from that which has caused the illness.

Basing on what is expressed above, the Applicant has found that the cigarettes whose filter contains the toxic substances of the cigarette yet in homeopathic concentration have a neutralizing effect for the damage from smoke.

Without wishing to be bound to any particular theory, it can be presumed that damage from smoke is reduced thanks to the fact that the user, when he or she smokes, is treated instantaneously with a dilution of the same toxic substances which cause intoxication thereof.

To this end reference should be made to the studies relating to inflammatory reactions performed during the Experiment of Doneghe, Experiment of Lallouette and of M. Aubin and Mme S. Baronnet, by means of which it was demonstrated that the taking of *apis* in homeopathic dilution allows the phenomenon of oedema caused by ultraviolet rays to be lessened provided it is taken immediately (isopathic method), basing on the reactive similarity which exists between the skin phenomena caused by the bee poison and the erythema caused by the exposure to ultraviolet rays.

In fact isopathy is a particular therapeutic procedure which consists in prescribing to a sick person a dilution, in general a medium-high one, of the substance responsible for the pathological disorders when there is a fully determined microbial or toxic cause.

The isopathic method already offers a first approach in order to demonstrate the action of the law of similarity: the principle is that of causing a controllable intoxication and therefore noting that which happens following administration at precise intervals of different dilutions of the same toxic product.

Among the many research projects carried out, some protocols which showed very significant results by demonstrating that a substance administered beforehand, diluted and dynamized homeopathically (by means of succussions), is likely in certain conditions to behave as antagonist of that same substance taken in a dose by weight.

The *neutralizing filter* of the present invention comes within this sphere (the *isopathic* one in fact).

In general it has to be pointed out that homeopathic dilutions generally have been studied for some time with pharmacological techniques and physical methods in order to specify their mechanisms of action. See for example E. Heintz, Strasbourg, "New experiments on the mode of action of successive dilutions" [Experimental testing of the law of Arndt & Schultz (in press)] and C. Luu De Vinh, "Homeopathic dilutions. Control and study with Laser Raman spectroscopy", doctorate dissertation, pharmacology, Montpellier 1974.

These studies relate to the physical and chemical structure of the homeopathic dilutions, and in particular the physical state of the Hahnemannian dilutions: they have demonstrated that the process of dynamization-succussion succeeds in modifying the solvent. This has been shown by means of Laser Raman spectrometry: by subjecting the homeopathic dilutions to a laser beam (monochromatic light beam) and adjusting to the Raman spectrograph the diffusions produced, particular spectra are obtained. These demonstrate that the characteristic waves of the solvent are found to be modified both as a function of the value of the dilutions and as a function of the substance diluted. It can therefore be admitted that it is this particular physical state which carries the reactive and therapeutic potential of a diluted and dynamized substance according to the teachings of Hahnemann.

"Homeopathic dilution" is understood to identify a deconcentration of a given substance in a vehicle and in order to do this homeopathy uses the mother tincture of said substance as starting point, performing a series of passages of dilution in an appropriate scale, for example decimal scales by means of a ratio 1:10 (decimal dilution) indicated by the letters D, DH, X, XH, centesimal scales by means of a ratio 1:100 (centesimal dilution) denoted by the letters C or CH (for the Hahnemannian dilutions) or K (Korsakovian dilutions), fifty millesimal scales, performing a dynamization every passage of dilution.

Dynamization is a process which involves a series of movements of succussions (shaking) of the dilution aimed at releasing the energy accumulated in the given substance to be rendered homeopathic: thanks to this process inert substances such as sand (Silicea) or oyster shell (Calcarea) can be used as a powerful medicine.

The starting homeopathic preparation used for the impregnation of the filter is a mother tincture (hydro-alcoholic solution) obtained by following the indications of the German homeopathic pharmacopeia and deriving from the maceration of paper and tobacco of the entire cigarette, but not the filter. Said mother tincture is subsequently subjected to the homeopathic procedure of dilution and dynamization until obtaining a predetermined homeopathic dilution of a degree normally used in the homeopathic preparations such as homeopathic dilutions D, CH, K, LM.

The effective dilutions for obtaining the result indicated above are preferably Hahnemannian centesimal dilutions CH or Korsakovian K, for example 30K, 200K, MK, XMK, or 15CH, 30CH, 100CH, 200CH, 1000 CH.

More preferably 200K dilutions are used (Korsakovian dilution).

Having obtained this homeopathic solution of a predetermined dilution, preferably 200K, the step begins of impregnation of normal filters for cigarettes with the same procedure used for the impregnation of homeopathic remedies in the form of granules or globules.

In general the solutions obtained from the dilution of a primary strain of substance are used for impregnating inert substrates according to the following ratios:
a) 1% volume/weight for the granules, globules and powder of lactose, where the volume is the quantity of solution in volume while the weight is the weight of the granule, globule and powder, i.e. 1 cc per 100 g;
b) 2% volume/weight for the tablets, i.e. 2cc of solution per 100g.

In the present case the inert substrate represented by the filter will be impregnated with the proportion indicated in point b), i.e. with 2cc of homeopathic solution with respect to the weight of the filter (100g).

The technique used for the impregnation of the filter according to the present invention is preferably an alternation of nebulisation and drying so as to ensure adsorption also at depth of the filter.

The impregnated filters are then inserted in the normal industrial production cycle of the cigarettes.

More particularly the present process for obtaining neutralized cigarettes consists of the following steps:

### Step 1:

Maceration of tobacco and paper of a cigarette of a predetermined composition, after shredding;

### Step 2:

Creation of a mother tincture from the maceration obtained in step 1;

### Step 3:

Creation of a homeopathic solution with predetermined dilution, preferably 200K, starting from said mother tincture by means of successive dilutions alternating with dynamizations (succussions);

### Step 4:

Impregnation of the cigarette filters of a predetermined composition with said homeopathic solution;

### Step 5:

Coupling of said soaked filters with said respective cigarettes to produce said cigarettes apt to neutralize the damage from smoke.

It should be noted that every type of cigarette to be "neutralized" will have its own mother tincture with which to impregnate its filters since its component elements such as mixture of tobacco and paper are different from brand to brand. A Lucky Strike mother tincture, a Pall Mall mother tincture and an MS mother tincture and so on will have to be produced.

The homeopathic neutralizing action takes place straightaway, while smoking, in that the homeopathic antidote is found in the filter, so that, when inhaling, the poisons and also their antidote are also inhaled simultaneously.

The present invention is an industrialisable homeopathic procedure which serves to neutralize the damage from smoke, not only caused by nicotine but also by all the other harmful substances contained in each cigarette.

This method can be applied to any cigarette of any brand, but the homeopathic process which neutralizes this damage should be repeated for each brand.

Moreover the soaking of the filter can be carried out with other concentrations different from the preferred 200K, such as for example 30K, MK, XMK, or 15CH, 30CH, 100CH, 200CH, 1000 CH, without thereby departing from the scope of the present invention.

The preferred homeopathic solution with 200K dilution of step 3 is obtained by means of the Korsakovian methodology which we will describe here for completeness of information.

The Korsakov method, also known as the single flask method, performs the dilution through successive operations in a liquid vehicle, specifically distilled water, operating in a single flask. It is the number of operations performed which defines the degree of dilution.

With this method liquid preparations are obtained, called Korsakovian dilutions, designated with the abbreviation K preceded by the number which corresponds to the degree of dilution.

Operatively the dilution is performed through the shaking of 5 ml of mother tincture in a 15 ml glass flask; the number of shakings (succussions) has to be 100 times, after which the flask is emptied through aspiration of the content; immediately afterwards 5 ml of distilled water are poured into the same flask, a quantity which represents 99 times the content of the mother tincture remaining on the walls of the flask, shaking is performed again for 100 times, thus obtaining the first Korsakovian dilution 1K. The process continues with the same method until reaching the required dilution.

Compared to that of Hahnemann, this method can be performed more simply, needs less material and solvent, is less wasteful and allows high homeopathic powers to be reached.

More particularly the homeopathic solution with 200K dilution of step 3 is obtained by means of a process known in the art of homeopathy for obtaining the mother tincture where the raw material constituted by paper and tobacco (not the filter, only the part which burns) is first shredded, then placed to macerate in a hydro-alcoholic solution as provided by the German homeopathy pharmacopeia (HAB) and subsequently filtered to separate the solution (mother tincture) from the remaining solid matter.

Having obtained the dilution required, preferably 200K (which also has the advantage of being easily industrialisable and in a simple manner) the step of impregnation of the cigarette filter (rather than the classic granules and/or globules) is carried out.

In practice the filters of the cigarettes to be neutralized can be impregnated with various systems used for the impregnation of the granules or of the globules, preferably with the system of so-called "triple impregnation" which provides for the alternating for 3 times of nebulisation and drying in order to ensure a deep penetration of the dilution in the heart of the filter.

The present method of soaking of the filter has to be repeated for each type of cigarette already in existence, in that each type of cigarette in existence has its own mother tincture with which to prepare the required dilution and impregnate in this way its filters.

The present method for obtaining a neutralized cigarette does not change absolutely the taste of the cigarette.

To date neutralized cigarettes with filter impregnated with homeopathic preparations are not known.

It is understood that what is described above can be applied also in the case wherein the homeopathic dilutions have a concentration different from 200K, for example 30K, MK, xMK, or 15CH, 30CH, 100CH, 200CH, 1000 CH.

The present invention is not limited to the particular embodiments previously described and illustrated in the accompanying drawings, but numerous detailed changes may be made thereto, within the reach of the person skilled in the art, without thereby departing from the scope of the invention itself as defined in the appended claims.

## Claims

1. Cigarette of a predetermined composition having a filter apt to neutralize damage from smoke, **characterised in that** said filter is soaked with a homeopathic preparation having a homeopathic dilution selected from D, CH, K or LM dilutions, said homeopathic preparation deriving from a mother tincture obtained from the maceration of paper and tobacco to be used for said cigarette, where the mixture of paper and tobacco constitutes the "homeopathic active ingredient".

2. Cigarette according to claim 1 wherein said filter is soaked with said homeopathic preparation through impregnation, preferably through the system of "triple impregnation".

3. Cigarette according to claim 1 or 2 wherein said homeopathic preparation is a 200K liquid homeopathic solution (Korsakovian dilution).

4. Cigarette according to any one of the preceding claims wherein said filter is soaked with 2% by volume of said homeopathic preparation with respect to the weight of the filter.

5. Process for preparing a cigarette neutralizing smoke damage as defined in the preceding claims 1 to 4, comprising the following steps:
Step 1:
Maceration of tobacco and paper of a cigarette of a predetermined composition, after shredding;
Step 2:
Creation of a mother tincture from the maceration obtained in step 1;
Step 3:
Creation of a homeopathic solution with a predetermined dilution, D, CH, K or LM, preferably 200K, starting from said mother tincture by means of successive dilutions alternating with succussions (shaking) so as to obtain dynamizations;
Step 4:
Impregnation of one or more cigarette filters of a predetermined composition with said homeopathic solution;
Step 5:
Coupling of said soaked filters with said respective cigarettes of said predetermined composition to produce said cigarettes apt to neutralize the damage from smoke.

6. Process according to claim 5 wherein the mother tincture of step 2 is obtained by chopping the tobacco and cigarette paper to obtain a shredded mixture, placing subsequently said shredded mixture to macerate in a hydro-alcoholic solution according to the German homeopathic pharmacopoeia, filtering the obtained solution to separate it from the solid material, obtaining said mother tincture.

7. Process according to claim 5 or 6 wherein the homeopathic solution with 200K dilution of step 3 is obtained by successive homeopathic dilutions of said mother tincture starting from a dilution thereof of 1:100 subjected to 100 movements of succussions to obtain the first Korsakovian centesimal dilution of 1K, repeating the dilution of 1:100 and the 100 succussions to obtain a second Korsakovian centesimal dilution of 2K, and so on up to 200K, using 99 parts distilled water as the dilution liquid.

8. Process according to any one of the preceding claims 5 to 7 wherein the soaking of the filters takes place by "triple impregnation."

## Patentansprüche

1. Zigarette mit einer vorbestimmten Zusammensetzung, die ein Filter aufweist, das geeignet ist, Schäden aus Rauch zu neutralisieren, **dadurch gekennzeichnet, dass** das Filter mit einer homöopathischen Zubereitung getränkt ist, die eine homöopathische Verdünnung aufweist, die aus D-, CH-, K- oder LM-Verdünnungen ausgewählt ist, wobei die homöopathische Zubereitung von einer Urtinktur stammt, die von der Mazeration von für die Zigarette zu verwendendem Papier und Tabak erhalten wird, wobei die Mischung von Papier und Tabak den "homöopathischen Wirkstoff" bildet.

2. Zigarette nach Anspruch 1, wobei das Filter mit der homöopathischen Zubereitung durch Imprägnierung, vorzugsweise durch das System der "Dreifach-Imprägnierung", getränkt wird.

3. Zigarette nach Anspruch 1 oder 2, wobei die homöopathische Zubereitung eine flüssige homöopathische 200K-Lösung (Korsakoff'sche Verdünnung) ist.

4. Zigarette nach einem der vorhergehenden Ansprüche, wobei das Filter mit 2 Vol.-% der homöopathischen Zubereitung bezogen auf das Gewicht des Filters getränkt ist.

5. Verfahren zum Herstellen einer Zigarette, die Schäden aus Rauch neutralisiert, nach den vorhergehenden Ansprüchen 1 bis 4, das die folgenden Schritte umfasst:
Schritt 1:
Mazeration von Tabak und Papier einer Zigarette mit einer vorbestimmten Zusammensetzung nach dem Zerkleinern;
Schritt 2:
Erzeugung einer Urtinktur aus der in Schritt 1 erhaltenen Mazeration;
Schritt 3:
Erzeugung einer homöopathischen Lösung mit einer vorbestimmten D-, CH-, K- oder LM-, vorzugsweise 200K-Verdünnung, ausgehend von der Urtinktur mittels aufeinanderfolgender Verdünnungen, die sich mit Sukkussionen (Rütteln) abwechseln, um Dynamisierungen zu erhalten;
Schritt 4:
Imprägnierung von einem oder mehreren Zigarettenfiltern mit einer vorbestimmten Zusammensetzung mit der homöopathischen Lösung;
Schritt 5:
Koppeln der getränkten Filter mit den entsprechenden Zigaretten mit der vorbestimmten Zusammensetzung zur Herstellung der Zigaretten, die geeignet sind, die Schäden aus Rauch zu neutralisieren.

6. Verfahren nach Anspruch 5, wobei die Urtinktur von Schritt 2 durch Zerhacken des Tabaks und Zigarettenpapiers, um eine zerkleinerte Mischung zu erhalten, anschließendes Platzieren der zerkleinerten Mischung zum Mazerieren in einer hydroalkoholischen Lösung gemäß dem Deutschen Homöopathischen Arzneibuch, Filtern der erhaltenen Lösung, um sie von dem Feststoff zu trennen, und Erhalten der Urtinktur erhalten wird.

7. Verfahren nach Anspruch 5 oder 6, wobei die homöopathische Lösung mit 200K-Verdünnung von Schritt 3 durch aufeinanderfolgende homöopathische Verdünnungen der Urtinktur ausgehend von einer Verdünnung davon von 1:100, die 100 Sukkussionsbewegungen unterzogen wird, um die erste Korsakoff'sche Centesimal-Verdünnung von 1K zu erhalten, Wiederholen der Verdünnung von 1:100 und der 100 Sukkussionen, um eine zweite Korsakoff'sche Centesimal-Verdünnung von 2K zu erhalten, und so weiter bis 200K unter Verwendung von 99 Teilen destilliertes Wasser als die Verdünnungsflüssigkeit erhalten wird.

8. Verfahren nach einem der vorhergehenden Ansprüche 5 bis 7, wobei das Tränken der Filter durch "Dreifach-Imprägnierung" erfolgt.

## Revendications

1. Cigarette d'une composition prédéterminée comportant un filtre apte à neutraliser les dommages dus à la fumée, **caractérisée en ce que** ledit filtre est imprégné avec une préparation homéopathique ayant une dilution homéopathique choisie parmi les dilutions D, CH, K ou LM, ladite préparation homéopathique dérivant d'une teinture mère obtenue à partir de la macération du papier et du tabac à utiliser pour ladite cigarette, où le mélange de papier et de tabac constitue le « principe actif homéopathique ».

2. Cigarette selon la revendication 1, dans laquelle ledit filtre est imprégné avec ladite préparation homéopathique par l'intermédiaire d'une imprégnation, de préférence par l'intermédiaire du système de « triple imprégnation ».

3. Cigarette selon la revendication 1 ou 2, dans laquelle ladite préparation homéopathique est une solution homéopathique liquide 200K (dilution korsakovienne).

4. Cigarette selon l'une quelconque des revendications précédentes, dans laquelle ledit filtre est imprégné avec 2 % en volume de ladite préparation homéopathique par rapport au poids du filtre.

5. Procédé de préparation d'une cigarette neutralisant les dommages de la fumée telle que défini dans les revendications précédentes 1 à 4, comprenant les étapes suivantes :
étape 1 :
la macération du tabac et du papier d'une cigarette d'une composition prédéterminée, après déchiquetage ;
étape 2 :
la création d'une teinture mère à partir de la macération obtenue dans l'étape 1 ;
étape 3 :
la création d'une solution homéopathique avec une dilution prédéterminée, D, CH, K ou LM, de préférence 200K, en démarrant à partir de ladite teinture mère au moyen de dilutions successives alternant avec des succussions (agitation) de façon à obtenir des dynamisations ;
étape 4 :
l'imprégnation d'un ou de plusieurs filtres de cigarette d'une composition prédéterminée avec ladite solution homéopathique ;
étape 5 :
le couplage desdits filtres imprégnés avec lesdites cigarettes respectives de ladite composition prédéterminée pour produire lesdites cigarettes aptes à neutraliser les dommages dus à la fumée.

6. Procédé selon la revendication 5, dans lequel la teinture mère de l'étape 2 est obtenue par le hachage du tabac et du papier de cigarette pour obtenir un mélange déchiqueté, le placement subséquent dudit mélange déchiqueté à macérer dans une solution hydro-alcoolique selon la pharmacopée homéopathique allemande, la filtration de la solution obtenue pour la séparer du matériau solide, l'obtention de ladite teinture mère.

7. Procédé selon la revendication 5 ou 6, dans lequel la solution homéopathique avec une dilution 200K de l'étape 3 est obtenue par des dilutions homéopathiques successives de ladite teinture mère en démarrant à partir d'une dilution de celle-ci de 1/100 soumise à 100 mouvements de succussions pour obtenir la première dilution centésimale korsakovienne de 1K, la répétition de la dilution de 1/100 et des 100 succussions pour obtenir une seconde dilution centésimale korsakovienne de 2K, et ainsi de suite jusqu'à 200K, en utilisant 99 parties d'eau distillée en tant que liquide de dilution.

8. Procédé selon l'une quelconque des revendications précédentes 5 à 7, dans lequel l'imprégnation des filtres s'effectue par « triple imprégnation ».
